# EUROPEAN PATENT APPLICATION

(11) **EP 3 138 572 A1**
(43) Date of publication of application: **08.03.2017**
(21) Application number: 16187147.0
(22) Date of filing: 02.09.2016
(51) Int. Cl.: A61K 35/76, C12N 7/00, C12N 9/36

(54) **ANTIMICROBIAL PEPTIDES DERIVED FROM PHAGE OF ACINETOBACTER BAUMANNII AND USE THEREOF**

(30) Priority: 04.09.2015 TW 104129310
(71) Applicant: Hualien Tzu Chi Hospital, Buddhist Tzu Chi Medical Foundation, Hualien County 97002 (TW)
(72) Inventor: Chen, Li-Kuang, Hualien City Hualien County 97002 (TW); Chang, Kai-Chih, Hualien City Hualien County 97002 (TW); Wu, Wen-Jui, Hualien City Hualien County 97002 (TW)
(74) Representative: Potter Clarkson LLP

(57) **Abstract**

The disclosure is related to antimicrobial peptides which are derived from the phage of *Acinetobacter baumannii.* The disclosure also provides antimicrobial compositions and methods of sterilizing microorganism *in vitro.*

## Description

### TECHNICAL FIELD

The present disclosure relates to a novel antimicrobial peptide, especially to an antimicrobial peptide derived from phage of *Acinetobacter baumannii.*

### BACKGROUND

### 1. Acinetobacter baumannii

*Acinetobacter baumannii* (AB) occurs broadly in natural environment of soil, water, food, etc., and also can be found on surface of normal human skin. *Acinetobacter baumannii* appears as red coccobacilli with a construction of capsule observed with optical microscope after Gram stain, belongs to non-fermenting Gram-negative coccobacilli in the term of biochemical properties, and is aerobic, non-motile and catalase-positive. *Acinetobacter baumannii* can grow in an environment at 42°C and can oxidize 10% lactose with optimum growth temperature of between 20°C to 30°C. Since *Acinetobacter baumannii* does not require particular growth conditions which can broadly occur on surface of various objects and in lifeless environment, its survival time can be more than 13 days and up to two weeks in a dry environment. Because of these advantages, the bacteria can survive long-term in dry or humid environment in hospital and becomes opportunistic nosocomial pathogen which rarely causes serious infection to healthy individuals and often causes opportunistic infection to long-term hospitalized patients with poor immunity. In 1998, for the first time, National Taiwan University Hospital isolated *Acinetobacter baumannii* having resistance to almost all antibiotics (pandrug-resistant *A. baumannii,* PDRAB). In addition to Taiwan, *Acifaetobacter baumannii* having resistance to various antibiotics, for example, beta-lactam antibiotics, aminoglycoside antibiotics, fluoroquinolone antibiotics and carbapenem antibiotics, has been isolated all over the world. It can be seen that *Acinetobacter baumannii* having resistance to multiple drugs has become a significant problem in Taiwan and abroad.

### 2. Phage

Bacteriophage (or phage) is a virus which can infect bacteria, similar to other viruses, consisting of nucleic acids and proteins as generic materials. The genetic material of a phage is injected to the bacteria during infection and is replicated using the enzymes of the host. Relevant proteins are assembled to form phages to be released from the bacteria to look for next hosts, when the survival environment is bad. The relevant proteins which are generated during the releases of phages to damage the bacteria include a lysozyme. The lysozyme can damage the cell wall of the bacteria by inducing the imbalance of osmotic pressure to break the bacteria.

### 3. Antimicrobial peptides

After the discovery of penicillin by Alexander Fleming, a British scientist, various antibiotics have been found and their chemical structures are modified and altered to increase therapeutic effects. In recent years, it becomes more and more difficult to treat drug-resistant bacterial strains due to the widely uses of antibiotics. New antibiotics have to be found to kill drug-resistant bacteria, and it is necessary to develop novel antimicrobial drugs.

In nature, numerous kinds of organisms can produce peptides having antimicrobial ability which can be used as the first line of defense of the congenital immune system against foreign pathogens. The peptides having antimicrobial ability commonly have following properties: (1) a length consisting of about 12 to about 100 amino acids; (2) charges of +2 to +9 valences; and (3) an amphipathic structure (with hydrophobic and hydrophilic regions). These properties of both being cationic and amphipathic enable the peptides to interact with the anion-carrying cell membranes or the phospholipid membranes of microorganisms more easily with the possibility of embedding into the membrane. Peptides having antimicrobial ability are present broadly in various organisms, such as melittins found in the venom of bee stings, pleurocidin (an antimicrobial peptide) found in vertebrates such as flatfish, and purothionins found in plants such as wheat seeds, and it is also found that the peptides having antimicrobial ability are even present in human neutrophils, monocyte and T lymphocyte.

Although there is such a rapid increase of drug-resistance in pathogenic microorganisms (e.g., *Acinetobacter baumannii*), the antimicrobial ability and range of the antimicrobial peptides which are naturally present or artificially modified are much less than that of the antibiotics. In order to avoid the risk of increasing the drug-resistance of pathogenic microorganisms due to the uses of antibiotics, it is an urgent task to find a method to kill the bacteria effectively without causing the drug-resistance of microorganisms.

### SUMMARY

The present disclosure provides an antimicrobial peptide derived from a lysozyme of *Acinetobacter baumannii* phage, wherein the phage is *Acinetobacter baumannii* phage No. 2 and deposited in Food Industry Research and Development Institute with the Accession No. BCRC 970047 and in Deutsche Sammlung von Mikroorganismen und Zellkulturen (DSMZ) with the Accession No. DSM 23600.

According to an aspect of the present disclosure, the antimicrobial peptide includes a modified sequence of SEQ ID No:1 and has lysozyme activity. In another aspect, the antimicrobial peptide has an amino acid sequence similarity of at least 72% to SEQ ID No.:1. In still another aspect, the modified sequence includes at least one of amino acid deletion and amino acid substitution in at least one positions of the SEQ ID No:1 selected from the group consisting of N113, P114, P120, I126, N133, G134, W135, G138, V139, G140, and F141.

According to an aspect of the present disclosure, the modified sequence includes the amino acid substitution of the SEQ ID No:1 in position P120. In still another aspect, the amino acid substitution is P120K.

According to an aspect of the present disclosure, the modified sequence includes the amino acid substitution of the SEQ ID No:1 in position I126. In still another aspect, the amino acid substitution is I126K.

According to an aspect of the present disclosure, the antimicrobial peptide has a hydrophobility of -0.34 to -1.26, an amphipathy of 0.29 to 0.47, and charges of +4 to +6.

According to an aspect of the present disclosure, the antimicrobial peptide has an amino acid sequence selected from the group consisting of SEQ ID No.:2, SEQ ID No.:3, SEQ ID No.:4 and SEQ ID No.:5.

In one aspect of the present disclosure, a bactericidal composition is also provided, which includes an antimicrobial peptide and a carrier thereof, wherein the antimicrobial peptide is derived from a lysozyme of *Acinetobacter baumannii* phage and the phage is deposited in Food Industry Research and Development Institute with the Accession No. BCRC 970047 and in Deutsche Sammlung von Mikroorganismen und Zellkulturen (DSMZ) with the Accession No. DSM 23600. According to an aspect of the present disclosure, the antimicrobial peptide is present in the bactericidal composition at a concentration substantially equal to or greater than about 4 µM.

In another aspect of the present disclosure, a sterilizing method is provided, which includes providing an antimicrobial peptide and subjecting the antimicrobial peptide to contact bacteria. According to an aspect, the antimicrobial peptide is in contact with the bacteria *in vitro.* In one aspect, the bacterium is *Acinetobacter baumannii,* and the antimicrobial peptide has the bactericidal ability against *Acinetobacter baumannii.*

### BRIEF DESCRIOPTION OF THE DRAWINGS

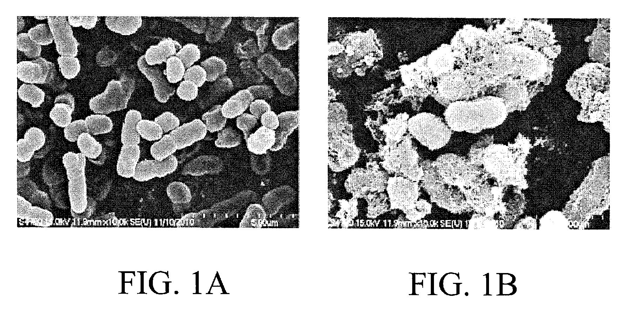

FIG. 1A and FIG. 1B are electron micrographs of bacteria showing the destruction of bacteria before and after the treatments of LysAB2-113-145, respectively.

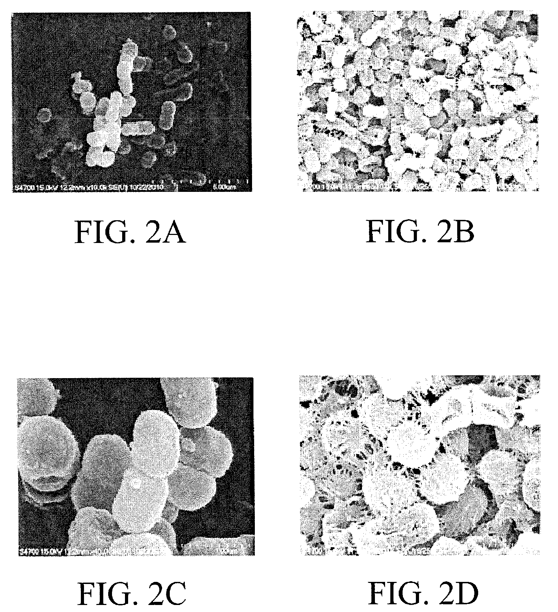

FIGs. 2A-2D are electron micrographs of bacteria showing the destruction of bacteria before and after the treatments of LysAB2 113-145 G4, of which FIG. 2A and FIG. 2B are electron micrographs showing the destruction of bacteria before and after the treatments of LysAB2 113-145 G4, respectively; FIG. 2C is a locally enlarged view of FIG. 2A; and FIG. 2D is a locally enlarged view of FIG. 2B.

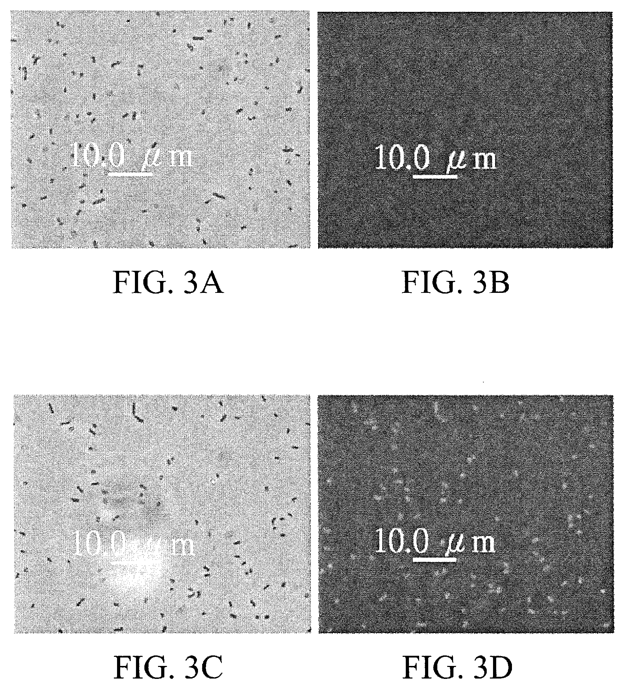

FIGs. 3A-3D are micrographs in bright field and under fluorescence, which show the situation of FITC entering bacteria after the destruction with LysAB2 113-145 G1, wherein FIG. 3A and FIG. 3B are micrographs of negative controls with the addition of phosphate buffer alone in bright field and under fluorescence, and FIG. 3C and FIG. 3D are micrographs in bright field and under fluorescence with the addition of LysAB2 113-145 G1 for 1 hour.

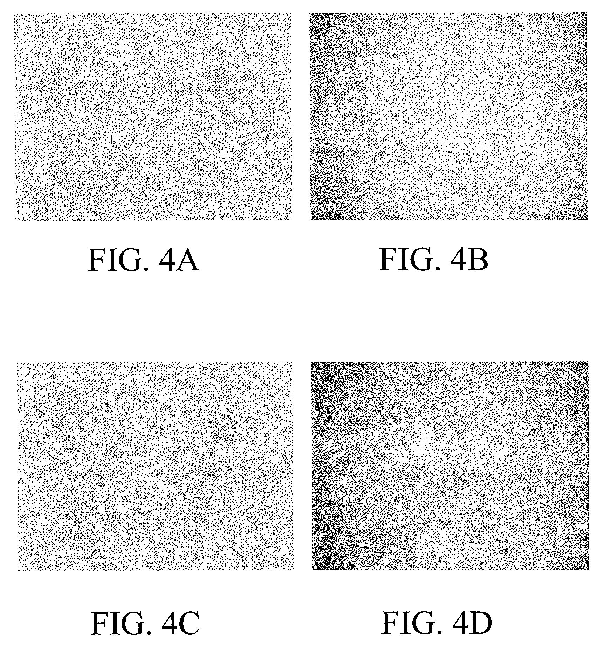

FIGs. 4A-4D are micrographs in bright field and under fluorescence, which show the situation of FITC entering bacteria after the destruction with LysAB2 113-145 G4, wherein FIG. 4A and FIG. 4B are micrographs in bright field and under fluorescence of negative controls with the addition of phosphate buffer alone, and FIG. 4C and FIG. 4D are micrographs in bright field and under fluorescence with the addition of LysAB2 113-145 G4 for 1 hour.

### DETAILED DESCRIPTION

Performance of the present disclosure is illustrated by following specific embodiments, and persons skilled in the art can understand other advantages and effects of the present disclosure based on the specification.

The present disclosure provides a peptide having antimicrobial ability, which is derived from a lysozyme of *Acinetobacter baumannii* phage. According to an aspect of the present disclosure, the phase is *Acinetobacter baumannii* phage No. 2 (BCRC 970047).

The term "peptide" used herein means a short chain containing more than one amino acid monomers, in which the more than one amino acid monomers are linked to each other by amide bonds. It must be noted that the amino acid monomers used in the peptide of the present disclosure are not limited to natural amino acids, and the amino acid sequence of the peptide can also include unnatural amino acids, compounds with similar structure, or the deficiency of amino acids.

The term "antimicrobial ability" used herein is evaluated using the results of minimal inhibitory concentration and minimum bactericidal concentration experiments, wherein the antimicrobial ability includes inhibitory and bactericidal activities

The term "structural parameters" used herein means the properties, for example, amphipathicity, conformation (e.g., α-helix and β-sheet), charge, polar angle, hydrophobicity and hydrophobic moment, which affect antimicrobial activity and selectivity. The structural parameters affect each other, and the change in one structural parameter generally results in the change of other parameters.

The term "amphipathicity" used herein indicates that one end of the peptide is hydrophobic and the other end is hydrophilic. The term "hydrophobic moment" used herein is a means for the measurement of amphipathic degree of a peptide, which can be presented by the sum of hydrophobic vector of each amino acid in the peptide.

In addition, the present disclosure provides a bactericidal composition comprising the antimicrobial peptide, and the composition can further include a carrier. Examples of the carrier are excipient, diluent, thickener, filler, binder, disintegrant, lubricant, lipid or non-lipid matrix, surfactant (e.g., Tween 20, Tween 80), suspending agent, gelling agent, adjuvant, preservative, anti-oxidant, stabilizing agent, colorant or fragrance.

The following specific embodiments are used to further explain features and effects of the present disclosure, but not to limit scope of the present disclosure.

### EAXMPLES

### Example 1. Design and amino acid sequence analysis of antimicrobial peptides

In the example, the carboxyl terminal of a lysozyme of *Acinetobacter baasmannii* phage No. 2 (deposited in Food Industry Research and Development Institute with the Accession No. BCRC 970047 and deposited in Deutsche Sammlung von Mikroorganismen und Zellkulturen (DSMZ) with the Accession No. DSM 23600) (hereinafter also called LysAB2) was used as the template for design of novel antimicrobial peptides. The carboxyl terminal region of LysAB2 is the No. 113 amino acid to the No. 145 amino acid (hereinafter also called LysAB2 113-145) and has a sequence showed by SEQ ID No.:1.

The carboxyl terminal structure of LysAB2 was analyzed by Multiple sequence alignment analysis software (clustalw_3D program in STRAP) (See, Gille C, Frommel C (2001) STRAP: editor for Structural alignments of proteins. Bioinformatics 17:377-378), and the result showed that the carboxyl terminal of LysAB2 was predicted to be a bipolar helix structure with a charge of +4, which may exhibit bactericidal activity.

Next, the structural parameters of LysAB2 113-145 peptide including peptide length, molecular weight, hydrophobicity, hydrophobic moment, amphipathicity (relative H. moment) and charge were modified to synthesize a new peptide having antimicrobial ability. Bioinformatics software including primary and secondary structure analysis software well known in the technical field were used in the modification of the structural parameters to predict the structural parameters.

Wherein, the primary structure analysis software can include but not limit to: ProtParam for the prediction of molecular weight of a peptide; and Pepstats for the analysis of charge distribution of a peptide. The secondary structure analysis software can include but not limit to: Jpred 3; PSIPRED Protein Predictor; and consensus hydrophobicity scale (CCS) for the analysis of hydrophobicity and hydrophobic moment. The software can be provided by Antimicrobial Peptide Database (APD).

Next, a screening was conducted on the modified peptides having the aforementioned structural parameters to obtain an antimicrobial peptide having antimicrobial ability. The peptides with modified sequences were synthesized by Mingxin BioTech Company. Four antimicrobial peptides derived from lysozyme of *Acinetobacter baumannii* phage were obtained and referred as LysAB2 113-145 G1, LysAB2 113-145 G2, LysAB2 113-145 G3 and LysAB2 113-145 G4, respectively, and their amino acid sequences were showed by SEQ ID No.:2 to 5, respectively.

The sequences and structural parameters of LysAB2 113-145 and the derived antimicrobial peptides are shown in Table 1.

**Table 1. Sequences and structural parameters of natural and derived antimicrobial peptides**

| Antimicrobial peptides | SEQ ID No: | Sequence | Length | Molecular Weight | Hydrophobicity | Amphipathicity | Charges |
|---|---|---|---|---|---|---|---|
| LysAB2 113-145 | 1 | -NPEKALEPLIAIQIAIKGMLNGWFTGVGFRR KR- | 33 | 3725.45 | -0.34 | 0.29 | +4.0 |
| LysAB2 113-145 G1 | 2 | ---EKALEKLIAIQKAIKGMLNGWFTGV-FRRK R- | 30 | 3503.25 | -0.99 | 0.47 | +6 |
| LysAB2 113-145 G2 | 3 | --EKALEPLIAIQIAIKGMLRAKFTA---RRKR- | 28 | 3365.17 | -1.26 | 0.29 | +6 |
| LysAB2 113-145 G3 | 4 | ---EKALEKLIAIQKAIKGMLAGWFTGV-ARRK R- | 30 | 3384.13 | -1.16 | 0.47 | +6 |
| LysAB2 113-145 G4 | 5 | -NPEKALEKLIAIQKAIKGMLNGWFTGVGFRR KR- | 33 | 3771.52 | -1.2 | 0.37 | +6.0 |

### Example 2. Inhibitory/bactericidal effects of the antimicrobial peptides

### Determination of Minimal Inhibitory Concentration (MIC)

In order to test the inhibitory effect of the antimicrobial peptides of the present disclosure, broth microdilution test was conducted to determine the minimal inhibition concentration (MIC) of the antimicrobial peptides against bacteria. The MIC represents the minimal concentration of the peptide which has the ability to inhibit bacteria growth completely.

The broth microdilution test were as following: selecting a single colony, inoculating in 3 mL of Muller-Hinton liquid medium (M-H broth, American BBL company, containing 2 g of beef extract powder, 17.5 g of acid hydrolyzed casein and 1.5 g of soluble starch per liter), culturing at 37°C for 2 to 6 hours; adjusting the bacterium suspension with M-H liquid medium to optical density OD₆₀₀ of 1 (1×10⁹ CFU/mL), diluting the bacterium suspension in 10-folds series to 5×10⁵ CFU/mL, taking 75 µL to a 96-well plate, then adding 75 µL of 256 µM peptide having antimicrobial ability, mixing for tens of times, then keeping the medium at 37°C, and observing the MIC result after culture in an incubator for 20 to 24 hours.

### Determination of Minimum Bactericidal Concentration (MBC)

In order to find out the minimum bactericidal concentration of the antimicrobial peptides of the present disclosure against bacteria, the surface of a suitable culture medium was covered by 100 µL of antimicrobial peptides at the minimal inhibitory concentration determined above with glass beads, then the medium was cultured at 37°C for 24 hours, thereafter, and observation was conducted for 3 days. When no colony grew, it is the indication that the bacteria have been killed. The minimal inhibitory concentration of the peptide which has the ability to completely inhibit the growth of the bacteria is the minimum bactericidal concentration.

*Acinetobacter baumannii* strains used in the examples in the conduction of the minimal inhibitory concentration and the minimum bactericidal concentration tests are shown in Table 2.

**Table 2. Acinetobacter baumannii strains being tested**

| Strains | Characteristics | Source |
|---|---|---|
| ATCC 17978 | Standard strains of *Acinetobacter baumannii* | American Type Culture Collection, ATCC |
| ATCC 17978CR | Colistin-resistant strain induced from ATCC 17978 | Induced by our team |
| ATCC 19606 | Standard strains of *Acinetobacter baumannii* | ATCC |
| ATCC 19606CR | Colistin-resistant strain induced from ATCC19606 | Induced by our team |
| BCRC 80276 | Multidrug-resistant *Acinetobacter baumannii* | Bioresource Collection and Research Center, BCRC |
| 39181 | Multidrug-resistant *Acinetobacter baumannii* | Taipei Tzuchi Hospital |
| 39400 | Multidrug-resistant *Acinetobacter baumannii* | Taipei Tzuchi Hospital |
| 44820 | Multidrug-resistant *Acinetobacter baumannii* | Taipei Tzuchi Hospital |

The minimal inhibitory concentration and the minimum bactericidal concentration results of the peptide having antibacterial ability provided according to some embodiments of the present disclosure are shown in Table 3.

**Table 3. Minimal Inhibitory Concentration (MIC) and Minimum Bactericidal Concentration (MBC) results of antimicrobial peptides against Acinetobacter baumannii**

| Antimicrobial peptides | SEQ ID No: | Inhibitory/Bactericidal Concentration* | *Acinetobacter baumannii* | | | | | | | |
|---|---|---|---|---|---|---|---|---|---|---|
| | | | ATCC 17978 | ATCC 17978CR^{A} | ATCC 19606 | ATCC 19606CR^{A} | BCRC 30276^{B} | 39181^{B} | 39400^{B} | 44820^{B} |
| LysAB2 | 1 | MIC | 64 | 64 | 64 | 64 | 64 | 64 | 64 | 64 |
| 113-145 | | MBC | 64 | 64 | 64 | 64 | 64 | 64 | 64 | 64 |
| LysAB2 | 2 | MIC | 8 | 16 | 8 | 16 | 8 | 8 | 8 | 8 |
| 113-145 G1 | | MBC | 8 | 16 | 8 | 16 | 8 | 8 | 8 | 8 |
| LysAB2 | 4 | MIC | 16 | 16 | 16 | 16 | 16 | 16 | 16 | 16 |
| 113-145 G3 | | MBC | 16 | 32 | 16 | 32 | 16 | 16 | 16 | 16 |
| LysAB2 | 5 | MIC | 4 | 8 | 4 | 8 | 4 | 4 | 4 | 4 |
| 113-145 G4 | | MBC | 4 | 8 | 4 | 8 | 4 | 4 | 4 | 4 |

| | | | | | | | | | | |
|---|---|---|---|---|---|---|---|---|---|---|
| * Concentration is represented in µM ^{A} *Acinetobacter baumannii* induced by our team which has drug-resistance against colistin ^{B} Multidrug-resistant *Acinetobacter baumannii* isolated by Taipei Tzuchi Hospital | | | | | | | | | | |

The results show that the antimicrobial peptides provided in the present disclosure can generate good inhibitory and bactericidal effects on either standard strains of *Acinetobacter baumannii* or multidrug-resistant *Acinetobacter baumannii* stains.

In addition, as shown in Table 3, LysAB2 113-145 has a minimal inhibitory concentration of 64 µM and a minimum bactericidal concentration of 64 µM. In contrast, after modification of structural parameters (e.g., charge, hydrophobicity and amphipathicity), the antimicrobial peptides derived from LysAB2 113-145 at a lower concentration can generate inhibitory and bactericidal effects on *Acinetobacter baumannii.* Among these, LysAB2 113-145 G4 has the best inhibitory and bactericidal effects on *Acinetobacter baumannii* with the minimal inhibitory concentration of 4 µM and the minimum bactericidal concentration of 4 µM, and demonstrates an excellent inhibitory/bactericidal effects (minimal inhibitory concentration of 4 µM; and minimum bactericidal concentration of 4 µM) on multidrug-resistant *Acinetobacter baumannii* in clinical application as well.

### Example 3. Scanning Electron Microscope

In order to observe whether the antimicrobial peptides of the present disclosure affect the appearance of bacteria, in reference with the experiment described by Mangoni, M. L. et al in "Effects of the antimicrobial peptide temporin L on cell morphology, membrane permeability and viability of Escherichia coli." (Biochem J(2004)380:859-65), 80 µM of antimicrobial peptides was added to a bacteria suspension containing 1×10⁹ CFU bacteria, and the destruction of bacterial was observed with scanning electron microscope after inoculation at 37°C for 1 hour.

The results are shown in FIGs. 1 and 2, wherein FIG. 1A is for the negative control group without treatment and FIG. 1B is that treated with LysAB2 113-145. It can be seen that *Acinetobacter baumannii* has a complete appearance and smooth surface in the negative control group without treatment of the antimicrobial peptides, i.e., the picture of *Acinetobacter baumannii* generally described as coccobacillus; while for *Acinetobacter baumannii* treated by the peptides having antibacterial ability as shown in FIG. 1B, it can be seen that the surface of the bacterial body is incomplete and has holes thereon, even is broken. In addition, the shape of *Acinetobacter baumannii* became nearly spherical shape from rod-shape, and there were many fragments around the bacterial body which were generated by broken bacteria. It is confirmed that antimicrobial peptides can cause incomplete outer structure and change in appearance of *Acinetobacter baumannii,* and further result in broken bacterial body.

FIG. 2A is the negative control group without treatment of LysAB2 113-145 G4, and FIG. 2B shows the bacteria treated with LysAB2 113-145 G4. FIG. 2C is the locally enlarged view of FIG. 2A, and FIG. 2D is the locally enlarged view of FIG. 2B. In comparing to the negative control group without treatment of antimicrobial peptides, the *Aciraetobacter baumannii* treated by the addition of LysAB2 113-145 G4 peptide having antibacterial ability has incomplete surface and holes thereon, and there are many fragments around the bacterial body, as a conclusion, the influence of LysAB2 113-145 G4 on the appearance of *Acinetobacter baumannii* body is more broad and clear than that of LysAB2 113-145.

### Example 4. Permeability Test of Antimicrobial Peptides on Bacterial Cell Membrane

In order to find out whether the antimicrobial peptides of the present disclosure cause permeability change on bacterial cell membrane, in reference with the method described by Mangoni M. L. et al. (2004), cells were stained with fluorescein isothiocyanate (FITC), and observation was conducted to confirm whether the antimicrobial peptides cause permeability change on bacterial cell membrane that allows FITC to enter the bacterial and to emit green fluorescence.

The operation steps were as following: culturing *Acinetobacter baumannii* in a LB liquid medium (from BioShop Canada, containing 5 g of NaCl, 10 g of tryptone, and 5 g of yeast extract per liter) at 37°C; centrifuging the liquid medium and washing and re-dissolving the cell pellet with phosphate buffer solution (PBS), when OD₆₀₀ of the liquid medium was greater than or equal to 1; diluting in serial dilution to obtain bacterial concentration of 2×10⁹ CFU/mL, taking 50 µL of bacteria suspension and culturing with 50 µL of LysAB2 113-145 G1 at 37°C for 1 hour as the treated group; additionally, taking 50 µL of the bacteria suspension and mixing with the equal amount of PBS as the negative control group; next, mixing 1 mL of FITC solution (6 µg/mL in phosphate buffer solution, Sigma) with the treated group and the control group, respectively, dropping on a glass sheet, and standing at a temperature of 37°C for 1 hour; and observing the results with fluorescence microscopy.

FIGs. 3A-3D are micrographs in bright field and under fluorescence of bacteria treated with antimicrobial peptides, wherein FIG. 3A and FIG. 3B are results observed in bright field and under fluorescence of negative control respectively. As shown in FIG. 3A, location of bacterial body can be seen directly in bright field. FIG. 3B is obtained by observation with fluorescence microscope, the fact that no obvious green (i.e., the color of FITC) is found means that treating Acinetobacter baumannii with PBS cannot affect the bacterial cell membrane. Also, FIG. 3C and FIG. 3D are results of the treated groups with addition of LysAB2 113-145 G1 in bright field and under fluorescence. As shown in FIG. 3C, location of bacterial body can be seen directly in bright field. FIG. 3D is obtained by observation with fluorescence microscope and many bacterial bodies in fluorescent green are observed, thus, it can be seen that the peptides having antibacterial ability of the present disclosure can affect cell membrane of *Acinetobacter baumannii,* change its permeability, and allow FITC dye to enter bacterial bodies to make it generate green fluorescence.

FIGs. 4A-4D show micrographs in bright field and under fluorescence of bacteria treated with LysAB2 113-145 G4, wherein FIG. 4A and FIG. 4B are results observed in bright field and under fluorescence of negative control respectively. As shown in FIG. 4A, location of bacterial body can be seen directly in bright field. FIG. 4B is obtained by observation with fluorescence microscope, the fact that no obvious green (i.e., the color of FITC) is found means that treating *Acinetobacter baumannii* with PBS cannot affect the bacterial cell membrane. Also, FIG. 4C and FIG. 4D are results of the treated group with the addition of LysAB2 113-145 G4 in bright field and under fluorescence. As shown in FIG. 4C, location of bacterial body can be seen directly in bright field. FIG. 4D is obtained by observation with fluorescence microscope and many bacterial bodies in fluorescent green are observed, thus, it can be seen that the peptides having antibacterial ability of the present disclosure can affect cell membrane of *Acinetobacter baumannii,* change its permeability, and allow FITC dye to enter bacterial bodies to make it generate green fluorescence.

In conclusion, the antimicrobial peptides of the present disclosure derived by using the peptide sequence of lysozyme of *Acinetobacter baumannii* phage No.2 as template and modifying its structural parameters has an inhibitory/bactericidal activity, and has a better effect than lysozyme peptide of *Acinetobacter baumannii* phage No. 2 which is used as the template. In addition, it is confirmed by above results that the antimicrobial peptides provided in the present disclosure has antibacterial effect both standard strains of *Acinetobacter baumannii* and multidrug-resistant and clinically multidrug-resistant *Acinetobacter baumannii* strains.

The above examples are described for illustration of the mechanisms and effects of the present disclosure, but not intended to limit the present disclosure. Anyone skilled in the art can perform modification and alteration on above examples without departing from the spirit and scope of the present disclosure. Therefore, the rights of the present disclosure should fall into the range as listed in the accompanying

## Claims

1. An antimicrobial peptide derived from a lysozyme of *Acinetobacter baumannii* phage, wherein the phage is *Acinetobacter baumannii* phage No. 2 and is deposited in Deutsche Sammlung von Mikroorganismen und Zellkulturen (DSMZ) with the Accession No. DSM 23600.

2. The antimicrobial peptide according to claim 1 including a modified sequence of SEQ ID No:1 and having a lysozyme activity.

3. The antimicrobial peptide according to any of the preceding claims, wherein the modified sequence includes at least one of amino acid deletion and amino acid substitution in at least one position of the SEQ ID No:1 selected from the group consisting of N113, P114, P120, I126, N133, G134, W135, G138, V139, G140, and F141.

4. The antimicrobial peptide according to any of the preceding claims, wherein the modified sequence includes the amino acid substitution of the SEQ ID No:1 in position P120 or I126.

5. The antimicrobial peptide according to any of the preceding claims, wherein the amino acid substitution is P120K or I126K.

6. The antimicrobial peptide according to any of the preceding claims having an amino acid similarity of at least 72% to the SEQ ID No.: 1.

7. The antimicrobial peptide according to any of the preceding claims having an amino acid sequence selected from the group consisting of SEQ ID No.:2, SEQ ID No.:3, SEQ ID No.:4 and SEQ ID No.:5.

8. The antimicrobial peptide according to any of the preceding claims having a bactericidal ability against *Acinetobacter baumannii.*

9. A bactericidal composition comprising the antimicrobial peptide according to any of the preceding claims and a carrier thereof.

10. The bactericidal composition according to claim 9, wherein the carrier is one selected from the group consisting of an excipient, a diluent, a thickener, a filler, a binder, a disintegrant, a lubricant, a lipid or non-lipid matrix, a surfactant, a suspending agent, a gelling agent, an adjuvant, a preservative, an anti-oxidant, a stabilizing agent, a colorant, a fragrance and any combination thereof.

11. The bactericidal composition according to claim 9, wherein the antimicrobial peptide is present in the bactericidal composition at a concentration substantially equal to or greater than about 4 µM.

12. A sterilizing method, comprising:
providing the antimicrobial peptide according to any of claims 1-8; and
subjecting the antimicrobial peptide to contact bacteria.

13. The sterilizing method according to claim 12, wherein the antimicrobial peptide is in contact with the bacteria *in vitro.*

14. The sterilizing method according to claim 12, wherein the antimicrobial peptide changes cell membrane permeability of the bacterial.

15. The sterilizing method according to claim 12, wherein the bacteria is *Acinetobacter baunaannii.*


SEQUENCE LISTING

<110> Hualien Tzu Chi Hospital, Buddhist Tzu Chi Medical Foundation

<120> ANTIMICROBIAL PEPTIDES DERIVED FROM PHAGE OF ACINETOBACTER BAUMANNII
AND USE THEREOF

<130> LOUBP/61756EP

<150> 104129310
<151> 2015-09-04

<160> 5

<170> PatentIn version 3.5

<210> 1
<211> 33
<212> PRT
<213> PHAGE OF ACINETOBACTER BAUMANNII

<400> 1

Asn Pro Glu Lys Ala Leu Glu Pro Leu Ile Ala Ile Gln Ile Ala Ile
1               5                   10                  15

Lys Gly Met Leu Asn Gly Trp Phe Thr Gly Val Gly Phe Arg Arg Lys
            20                  25                  30

Arg

<210> 2
<211> 30
<212> PRT
<213> Artificial Sequence

<220>
<223> LysAB2 113-145 G1

<220>
<221> PEPTIDE
<222> (1)..(30)

<400> 2

Glu Lys Ala Leu Glu Lys Leu Ile Ala Ile Gln Lys Ala Ile Lys Gly
1               5                   10                  15

Met Leu Asn Gly Trp Phe Thr Gly Val Phe Arg Arg Lys Arg
            20                  25                  30

<210> 3
<211> 28
<212> PRT
<213> Artificial Sequence

<220>
<223>  LysAB2 113-145 G2


<220>
<221>  PEPTIDE
<222>  (1)..(28)

<400>  3

Glu Lys Ala Leu Glu Pro Leu Ile Ala Ile Gln Ile Ala Ile Lys Gly
1               5                   10                  15


Met Leu Arg Ala Lys Phe Thr Ala Arg Arg Lys Arg
            20                  25


<210>  4
<211>  30
<212>  PRT
<213>  Artificial Sequence

<220>
<223>  LysAB2 113-145 G3


<220>
<221>  PEPTIDE
<222>  (1)..(30)

<400>  4

Glu Lys Ala Leu Glu Lys Leu Ile Ala Ile Gln Lys Ala Ile Lys Gly
1               5                   10                  15


Met Leu Ala Gly Trp Phe Thr Gly Val Ala Arg Arg Lys Arg
            20                  25                  30


<210>  5
<211>  33
<212>  PRT
<213>  Artificial Sequence

<220>
<223>  LysAB2 113-145 G4


<220>
<221>  PEPTIDE
<222>  (1)..(33)

<400>  5

Asn Pro Glu Lys Ala Leu Glu Lys Leu Ile Ala Ile Gln Lys Ala Ile
1               5                   10                  15


Lys Gly Met Leu Asn Gly Trp Phe Thr Gly Val Gly Phe Arg Arg Lys
            20                  25                  30

Arg